# EUROPEAN PATENT SPECIFICATION

(11) **EP 3 562 476 B1**
(45) Date of publication and mention of the grant of the patent: **11.05.2022**
(21) Application number: 16840322.8
(22) Date of filing: 30.12.2016
(51) Int. Cl.: A61K 9/51, A61K 31/352

(54) **A PRODUCT FOR PREVENTING AND RECOVERING OXIDATIVE DAMAGE IN LIVING CELLS**
PRODUKT ZUR VERHINDERUNG UND WIEDERHERSTELLUNG NACH OXIDATIVEN SCHÄDEN IN LEBENDEN ZELLEN
PRODUIT DE PRÉVENTION ET RÉCUPÉRATION DES DOMMAGES OXYDATIFS DANS DES CELLULES VIVANTES

(43) Date of publication of application: 06.11.2019
(73) Proprietor: Sabanci Universitesi, 34956 Istanbul (TR)
(72) Inventor: BASAGA, Huveyda, 34956 Tuzla, Istanbul (TR); BIRINCI, Yelda, 34956 Tuzla, Istanbul (TR)
(74) Representative: Sevinç, Erkan
(86) International application number: PCT/TR2016/050577
(87) International publication number: WO 2018/124994

(56) References cited:
- QIU DONGFANG ET AL: "Quercetin-sensitized TiO2film: Photocatalytic inhibition ofHelminthosporium maydisunder visible light irradiation", MATERIALS LETTERS, ELSEVIER, AMSTERDAM, NL, vol. 97, 25 January 2013 (2013-01-25), pages 21-23, XP028999409, ISSN: 0167-577X, DOI: 10.1016/J.MATLET.2013.01.089
- ZDYB AGATA ET AL: "Characterization of adsorption and electronic excited states of quercetin on titanium dioxide nanoparticles", SPECTROCHIMICA ACTA. PART A: MOLECULAR AND BIOMOLECULAR SPECTROSCOPY, ELSEVIER, AMSTERDAM, NL, vol. 157, 7 January 2016 (2016-01-07), pages 197-203, XP029393308, ISSN: 1386-1425, DOI: 10.1016/J.SAA.2016.01.006

## Description

### Technical Field of the Invention

This invention is related to a product for protection against oxidative stress and recovering oxidative damage in living cells.

### Prior Art

Recently, it is well known that the oxidative stress plays an important role for many diseases and pathological conditions such as cancer, neurodegenerative diseases, cardiovascular diseases and aging. Oxidative stress occurs when the antioxidant defense of the cell is inadequate against reactive oxygen species (ROS). This makes exogenous "antioxidant therapy" a promising strategy for supporting cell antioxidant defense for preventing many diseases caused by oxidative damage.

Quercetin is one of the members of flavonoids which are phenolic phytochemical compounds present in high amounts in natural resources. It has unique biological and pharmacological properties such as antioxidant, anti-carcinogenic, anti-inflammatory, antihypertensive, antiviral and antibacterial activities. In particular; quercetin is the most powerful flavonoid with the high-efficiently antioxidant effect, categorized by various unique properties such as oxygen radical scavenging, avoiding of lipid peroxidation, metal ion chelation and regulating of cell antioxidant responses. However, quercetin application is very inefficient because of its poorly water-soluble nature. At this point, it requires an efficient vehicle for overcoming that of its limited application.

Nanoparticles are particularly useful in drug delivery for water-insoluble molecules such as chemotherapeutic drugs. In the case of nanoparticles of TiO₂; it is approved by FDA (US food and drug administration) as a biological inert component in medical field, it has been widely used in sunscreen products as a major substance against high ultraviolet rays in cosmetics due to its high-efficiently photoactive properties. Other common usages are orthopedic and dental implants.

Quercetin was formulated in various forms such as nanoformulations, liposomes, glycerosomes for overcoming less water solubility and increase its stability (Hatahet T, Morille M, Hommoss A, Devoisselle JM, Muller Begu RH. Quercetin topical application, from conventional dosage forms to nanodosage forms. Eur J Pharm Biopharm. Aug 23, 2016).

In the state of the art, the paper titled as **"**Quercetin deformable liposome: preparation and efficacy against ultraviolet B induced skin damages in vitro and in vivo" (Liu et al., 2013) high solubility and better stability of deformable liposomes incorporated with quercetin improved skin penetration in HaCat cells.

The paper titled as "Beneficial effects of quercetin on oxidative stress in liver and kidney induced by titanium dioxide (TiO2) nanoparticles in rats" (Aarón Emmanuel Gonzalez-Esquivel et al., 2015) established antioxidative effect of quercetin treatment against oxidative stress induced by TiO₂ nanoparticles.

The paper titled as "Quercetin-sensitized TiO2 film: Photocatalytic inhibition of Helminthosporium maydis under visible light irradiation" (Dongfang et al., Materials letters 97 (2013) 21-23) discloses a TiO₂ film prepared by PEG-added sol-gel method via a dip-coating technique.

### Brief Description of the Invention

The aim of the invention is to maximize the antioxidant effect of quercetin for prevention or recovery of the oxidative stress in living cells.

Another aim of the invention is to improve quercetin penetration into cells.

In this invention; "nano" is defined as having particle size equal to or less than 100 nm.

The disclosed invention comprises antioxidant molecules loaded to at least one nanoparticle. Preferably, it comprises the nanoparticle encapsulated with a predetermined material for surface modification.

### Detailed Description of the Invention

The present invention provides one or more TiO₂ nanoparticles according to claim 1 and a method for obtaining TiO₂ nanoparticles according to claim 9.

The disclosed invention is quercetin loaded TiO₂ nanoparticle in which the quercetin is an antioxidant and TiO₂ nanoparticle is a nanocarrier for quercetin. Usage of TiO₂ nanoparticle increases quercetin penetration to the cell because of its high biosafety, antibacterial and inert properties.

The invention comprises TiO₂ nanoparticle encapsulated with a predetermined material and quercetin which is loaded to said encapsulated TiO₂ nanoparticle. In the preferred embodiment; the predetermined material is polyethylene glycol (PEG), more preferably PEG having average molecular weight 6000 g/mol.

The encapsulated TiO₂ nanoparticle provides surface modification before loading quercetin. Therefore, the efficiency of the invention increases because the nanoparticle has a more adhesive surface and so the antioxidant molecules are adhered to the surface of the nanoparticle. PEG used as predetermined material in the preferred embodiment increases the biocompatibility of TiO₂ nanoparticle and makes the surface of TiO₂ nanoparticle more adhesive. Therefore; quercetin loading ratio (quercetin loaded surface area of the nanoparticle/whole surface area) is increased because of the adhesiveness of the TiO₂ nanoparticle surface. This ratio reaches up to 95%.

Loading quercetin to TiO₂ nanoparticle provides that keeping the high concentration quercetin is stable and functional without any cytotoxicity in cell and by this way it is able to show a strong antioxidant activity against to ROS.

The disclosed quercetin loaded TiO₂ nanoparticle has maximum antioxidant capacity (its functional capacity, 100%), maximum biocompatibility and no cytotoxicity, maximum loading ratio of quercetin because of provided quercetin solubility and encapsulation of nanoparticles, maximum stability of quercetin in cellular environment.

The disclosed quercetin loaded TiO₂ nanoparticle may be used as a dermatological preparate topically.

## Claims

1. One or more TiO₂ nanoparticles for penetration into living cells as a nanocarrier, said TiO₂ nanoparticles having a particle size of up to 100 nm, which are
- surface modified with polyethylene glycol (PEG) having average molecular weight of 6000 g/mol, and
- loaded with quercetin.

2. A dermatological preparate for topical use, comprising the TiO₂ nanoparticles according to the claim 1.

3. A medicament comprising the TiO₂ nanoparticles according to the claim 1.

4. The medicament according to the claim 3, for use in treatment of neurogenerative diseases.

5. The medicament according to the claim 3, for use in treatment of cancer.

6. The medicament according to the claim 3, for use in treatment of cardiovascular diseases.

7. A cosmetic product comprising the TiO₂ nanoparticles according to the claim 1.

8. An anti-aging product comprising the TiO₂ nanoparticles according to the claim 1.

9. A method for obtaining TiO₂ nanoparticles for penetration into living cells as a nanocarrier, said TiO₂ nanoparticles having a particle size of up to 100 nm, which are encapsulated with polyethylene glycol (PEG) having average molecular weight of 6000 g/mol, and loaded with quercetin; said method including
- surface modification of TiO₂ nanoparticles having a particle size of up to 100 nm with PEG having average molecular weight of 6000 g/mol, followed by
- loading said TiO₂ nanoparticles with quercetin.

## Patentansprüche

1. Ein oder mehrere TiO₂-Nanopartikel zum Eindringen in lebende Zellen als Nanocarrier, wobei die TiO₂-Nanopartikel eine Partikelgröße von bis zu 100 nm besitzen, die
- mit Polyethylenglycol (PEG) mit einem durchschnittlichen Molekulargewicht von 6000 g/mol oberflächenmodifiziert sind, und
- mit Quercetin angereichert sind.

2. Dermatologisches Präparat zur topischen Anwendung, das die TiO₂-Nanopartikel nach Anspruch 1 umfasst.

3. Medikament, das die TiO₂-Nanopartikel nach Anspruch 1 umfasst.

4. Medikament nach Anspruch 3, zur Verwendung bei der Behandlung neurodegenerativer Erkrankungen.

5. Medikament nach Anspruch 3, zur Verwendung bei der Behandlung von Krebs.

6. Medikament nach Anspruch 3, zur Verwendung bei der Behandlung Herz-Kreislauf-Erkrankungen.

7. Kosmetisches Produkt, das die TiO₂-Nanopartikel nach Anspruch 1 umfasst.

8. Anti-Aging-Produkt, das die TiO₂-Nanopartikel nach Anspruch 1 umfasst.

9. Verfahren zur Gewinnung von TiO₂-Nanopartikeln zum Eindringen in lebende Zellen als Nanocarrier, wobei die TiO₂-Nanopartikel eine Partikelgröße von bis zu 100 nm besitzen, die mit Polyethylenglycol (PEG) mit einem durchschnittlichen Molekulargewicht von 6000 g/mol verkapselt sind und mit Quercetin angereichert sind; wobei das Verfahren die folgenden Schritte umfasst:
- Oberflächenmodifikation von TiO₂-Nanopartikeln mit einer Partikelgröße von bis zu 100 nm mit PEG, das ein durchschnittliches Molekulargewicht von 6000 g/mol besitzt, und anschließend
- Anreichern der TiO₂-Nanopartikel mit Quercetin.

## Revendications

1. - Une ou plusieurs nanoparticules de TiO₂ pour la pénétration dans des cellules vivantes en tant que nanosupport, lesdites nanoparticules de TiO₂ ayant une dimension de particule allant jusqu'à 100 nm, qui sont
- modifiées en surface par du polyéthylène glycol (PEG) ayant une masse moléculaire moyenne de 6000 g/mol ; et
- chargées en quercétine.

2. - Préparation dermatologique à usage topique, comprenant les nanoparticules de TiO₂ selon la revendication 1.

3. - Médicament comprenant les nanoparticules de TiO₂ selon la revendication 1.

4. - Médicament selon la revendication 3, destiné à être utilisé dans le traitement de maladies neurodégénératives.

5. - Médicament selon la revendication 3, destiné à être utilisé dans le traitement de cancer.

6. - Médicament selon la revendication 3, destiné à être utilisé dans le traitement de maladies cardiovasculaires.

7. - Produit cosmétique comprenant les nanoparticules de TiO₂ selon la revendication 1.

8. - Produit anti-âge comprenant les nanoparticules de TiO₂ selon la revendication 1.

9. - Procédé d'obtention de nanoparticules de TiO₂ pour la pénétration dans des cellules vivantes en tant que nanosupport, lesdites nanoparticules de TiO₂ ayant une dimension de particule allant jusqu'à 100 nm, qui sont encapsulées par du polyéthylène glycol (PEG) ayant une masse moléculaire moyenne de 6000 g/mol, et chargées en quercétine ; ledit procédé comprenant :
- une modification de surface de nanoparticules de TiO₂ ayant une dimension de particule allant jusqu'à 100 nm par du PEG ayant une masse moléculaire moyenne de 6000 g/mol, suivie par
- un chargement desdites nanoparticules de TiO₂ en quercétine.
